# EUROPEAN PATENT APPLICATION

(11) **EP 2 002 782 A2**
(43) Date of publication of application: **17.12.2008**
(21) Application number: 07740967.0
(22) Date of filing: 04.04.2007
(51) Int. Cl.: A61B 5/00, G06Q 50/00

(54) **MEDICAL INFORMATION PROCESSING DEVICE**

(30) Priority: 06.04.2006 JP 2006105321
(71) Applicant: Konica Minolta Medical & Graphic, Inc., Hino-shi, Tokyo 191-8511 (JP)
(72) Inventor: SASANO, Yasuhiko, Hachioji-shi, Tokyo 192-8505 (JP)
(74) Representative: Gille Hrabal Struck Neidlein Prop Roos
(86) International application number: PCT/JP2007/057531
(87) International publication number: WO 2007/116898

(57) **Abstract**

To make it possible to display a series of image data captured according to examination order information, in a predetermined order. According to an image examination device (3) as the medical information processing device, image data received from modality as an image generation device is classified by examination order information according to the examination ID. Examination information contained in the additional information on each image data captured according to the same examination order information is acquired. Each of the acquired examination information is subjected to code conversion according to a display conversion table (361) and the converted codes are sorted in the ascending order. An image examination waiting list number is assigned to each image data in the order corresponding to the sort result and successively added and registered in an image examination waiting image storage section (362). Additional information on the image data stored in the image examination waiting image storage section (362) is arranged in a list according to the image examination waiting list number and the list is displayed on a display section (33).

## Description

### Technical Field

The present invention relates to a medical information processing device and in particular, a medical information processing device (hereinafter referred to as, image examination device) to display a medical image generated by an image generation device based on examination order information and supplementary information, so that an image examiner may confirm and modify the medical image and the supplementary information thereof.

### Background Art

In the field of medicine, digitalization of medical images of patients is realized, and a medical image system is being used, where image data of medical images photographed and digitalized by various image generation devices (modality) such as Computed Radiography (CR) device, Computed Tomography (CT) device and MRI (Magnetic Resonance Imaging) device is stored in a Picture Archiving and Communication System for medical application (PACS) database with supplementary information such as information of a patient to be examined (photographed) or examination information (information of modality used in the examination, information of photographed site, information of photographed direction, date of photography, etc.) or the image data is displayed on an image display device for diagnosis by a doctor.

Typically, image data generated in the modality and received by PACS or the image display device is stored in the image database or displayed on the image display device in an order the image data is received. However, the order the image data is received is not necessarily a display order which one who accesses to the medical image can work with efficiently.

Thus, for example, patent document 1 describes a technique where display status of images collected in a series of photographing using an MRI device or CT device is changed.
Patent Document 1: Japanese Patent Application Laid-Open Publication No. 2004-337347

### Disclosure of the Invention

### Problems to be Solved by the Invention

Photographing using modalities is performed according to examination order information registered in a Radiological Information System (RIS) or Hospital Information System (HIS). Typically, examination order information is registered for a series of examinations performed to a same patient on a same day. A pattern of a series of examinations is fixed to some extent in each medical facility according to examination site or case, however in some examination patterns, a plurality of examination information with a different combination of modality, site of photography, direction of photography, etc., may be included in one examination order information.

Thus, even if the series of examinations are patterned, at the examination (photography) location, there are many occasions where a patient comes and goes between a plurality of modalities or a plurality of modalities photograph a plurality of patients at the same time, and when the images are displayed in the order the image data is received, even if a series of medical images are photographed according to a predetermined examination pattern, the image display order is not in a predetermined order, and images of different patients may be mixed.

In recent years, an image examination device is proposed, the device receiving examination order information from RIS and HIS as well as receiving image data from a modality in order to check whether a series of images in one examination order information is photographed without omission, consistency between examination order information and supplementary information, or quality of the generated image, however, as described above, if the images are displayed in the order the image data is received, even if a series of medical images are photographed according to a predetermined examination pattern, the image display order is not in a predetermined order, and images of different patients may be mixed, thus the image examiner needs to change the order of the images which is troublesome.

In the above-described technique of patent document 1, the display order of image data is specified and changed according to modality, thus even by using this technique, the above-described problem of display order of a series of images photographed according to examination order information cannot be solved.

An object of the present invention is to enable a series of image data photographed according to examination order information to be displayed in a predetermined order.

### Means for Solving the Problem

In order to achieve the above object, according to a first aspect of the present invention, there is provided a medical information processing device connected to one or more image generation device which generates a medical image based on examination order information and supplements to the generated medical image supplementary information including identification information for identifying the examination order information, and patient information and examination information concerning the medical image, the medical information processing device including a display section to display the medical image generated by the image generation device, the medical information processing device comprising:
a receiving section to receive the medical image generated by the one or more image generation device and to receive the supplementary information of the medical image;
a sorting section to classify the medical image received by the receiving section with respect to medical image generated based on the examination order information and to sort the examination information supplemented to the medical image generated based on a same examination order information in a predetermined order; and
a control section to allow the display section to display a series of the medical image generated based on the same examination order information by the one or more image generation device or to display a list of the series of medical image.

Preferably, the medical information processing device further comprises:
a conversion table storage section which stores a conversion table for converting the examination information into a code representing the examination information; and
a code conversion section to convert the examination information supplemented to the medical image generated based on the same examination order information according to the conversion table, wherein
the sorting section sorts the examination information by sorting in ascending order the code resulting from the conversion by the code conversion section.

Preferably, in the medical information processing device,
the examination information includes image generation device information, photograph site information, photograph direction information, body position information and left and right information to specify content of the examination;
the medical information processing device includes a sorting order table storage section to store a table showing a sorting order of the image generation device information, a table showing a sorting order of the photograph site information, a table showing a sorting order of a combination of the photograph direction information, body position information and left and right information; and
the sorting section sorts the examination information supplemented to the medical image generated based on the same examination order information according to the table stored in the sorting order table storage section.

### Advantageous Effect of the Invention

According to a first aspect of the present invention, a series of medical images according to examination order information and a list of the images are displayed in a predetermined order, thus an image or list of a series of medical images photographed in a predetermined examination pattern is constantly displayed in a predetermined order and an operator performing a check by displaying the photographed medical image can easily know whether or not all of the examinations in one examination order information are performed without changing the display order of the medical image or list and can easily check the series of images in a predetermined order, thus operating efficiency can be enhanced.

### Brief Description of the Drawings

FIG. 1 is a diagram showing an example of an overall structure of a medical image system of an embodiment of the present invention;
FIG. 2 is a block diagram showing a functional structure of an image examination device shown in FIG. 1;
FIG. 3 is a diagram showing a data structure of JJ1017 Ver. 3.0 code;
FIG. 4 is a diagram showing an example of a data structure of a display conversion table stored in a storage section shown in FIG. 2;
FIG. 5A is a flow diagram showing processing and flow of data in devices configuring the medical image system shown in FIG. 1;
FIG. 5B is a flow diagram showing processing and flow of data in devices configuring the medical image system shown in FIG. 1;
FIG. 6 is a flow chart showing image display order rearranging processing performed by a CPU shown in FIG. 2;
FIG. 7 is a diagram showing an example of an image examination waiting list displayed on a display section shown in FIG. 2;
FIG. 8A is a diagram showing an example of a data structure of a display conversion table stored in a storage section shown in FIG. 2;
FIG. 8B is a diagram showing an example of a data structure of a modality display order table 364a shown in FIG. 8A;
FIG. 8C is a diagram showing an example of a data structure of a site display order table 364b shown in FIG. 8A;
FIG. 8D is a diagram showing an example of a data structure of a direction display order table 364c shown in FIG. 8A; and
FIG. 9 is a flow chart showing image display order rearranging processing B performed by the CPU shown in FIG. 2.

### Best Mode for Carrying Out the Invention

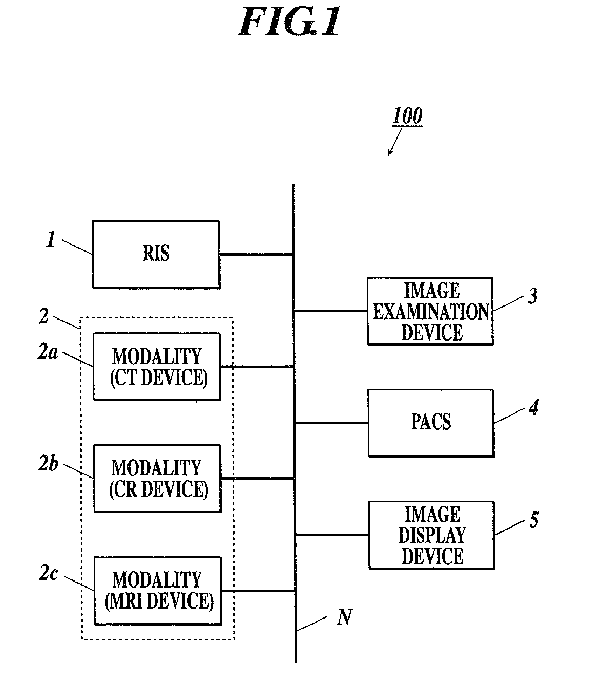

An embodiment of a medical image system of the present invention will be described below with reference to FIG. 1 to FIG. 9. However, the present invention is not limited to the illustrated examples.

### [First Embodiment]

First, a structure of the first embodiment will be described.

FIG. 1 shows a system structure of a medical image system 100 of the first embodiment.

As shown in FIG. 1, the medical image system 100 includes RIS 1, modalities 2 (2a to 2c), an image examination device 3, PACS 4 and an image display device 5 connected through a communication network N such as a Local Area Network (LAN), Wide Area Network (WAN) and the like so that data can be sent and received among each other.

The devices configuring the medical image system 100 will be described below.

RIS 1 is a radiological information system for managing information in a radiological department and the system receives registration of examination order information (for example, patient information for identifying the patient to be examined, such as patient ID, name, sex, etc., or examination information, such as information of modality used in examination (photography) of a medical image (for example, CT, CR, MRI, etc.), photograph method (procedure) information (for example, X-ray plain photography, X-ray fluoroscopic examination, etc.), photograph site information (for example, chest, abdomen, etc.), photograph direction information (for example, front, side, etc.), body position information (for example, upright, recumbent, etc.), left and right information (for example, right, left, etc.) and the like) from an operator to generate examination order information, and an examination ID which is identification information of the examination order information is attached to the generated examination order information to manage the information. The examination order information includes patient information of a patient and examination information of one or a plurality of relating examinations. For example, when an examination performed on a patient is only examination of photographing front chest in recumbent position with the CR device 2b, the examination information included in the examination order information is one. When examinations performed on a patient are photography of front chest in recumbent position with the CR device 2b and photography of chest in recumbent position with the CT device 2a, the examination information included in the examination order information is two. The RIS 1 sends the generated examination order information to the modality 2 and the image examination device 3 specified by the modality information of the examination order information.

In the present embodiment, the examination order information generated by the RIS 1 is sent to the modality 2 and the image examination device 3, however, a reception terminal which performs reception for issuing of examination order information may generate examination order information and send the information to the modality 2.

The modality 2 is an image generation device for photographing a patient to generate image data of a medical image. In the present embodiment, the medical image system 100 includes the CT device 2a, CR device 2b, and MRI device 2c as modalities 2, however, various types of modalities for photographing a medical image can be used such as an endoscopic device, ultrasonic diagnostic device, etc.

The modality 2 photographs a patient and generates image data of the medical image according to examination order information generated by the RIS 1. Then, examination ID of examination order information corresponding to the medical image received from the RIS 1 and among the examination order information, information including patient information and examination information for identifying the medical image is supplemented to the generated image data of the medical image as supplementary information and sent to the image examination device 3.

The image data and the supplementary information sent from the modality 2 to the image examination device 3 are sent for example as a DICOM file including an image section and a header section. The image data is written in the image section and supplementary information (examination ID, patient information, examination information, etc.) concerning the image data is written in the header section.

The image examination device 3 is a medical information processing device which receives examination order information sent from the RIS 1 as well as the image data of the medical image generated by the modality 2 and displays the received medical image and supplementary information on the display section 33 to allow the image examiner to check and modify the medical image and the supplementary information through the input section 32.

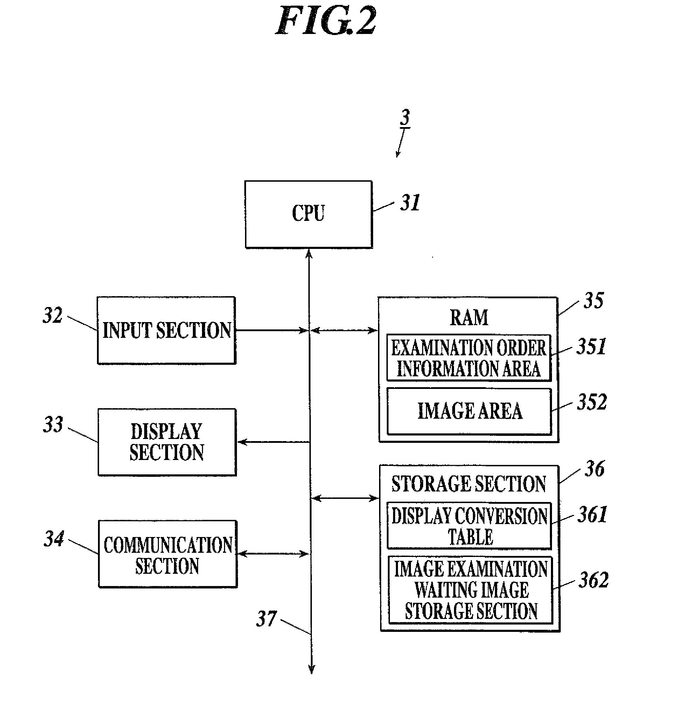

Fig. 2 shows an inner structure of the image examination device 3.

As shown in Fig. 2, the image examination device 3 includes a CPU 31, an input section 32, a display section 33, a communication section 34, a RAM 35, a storage section 36, etc., and the sections are connected to each other by a bus 37.

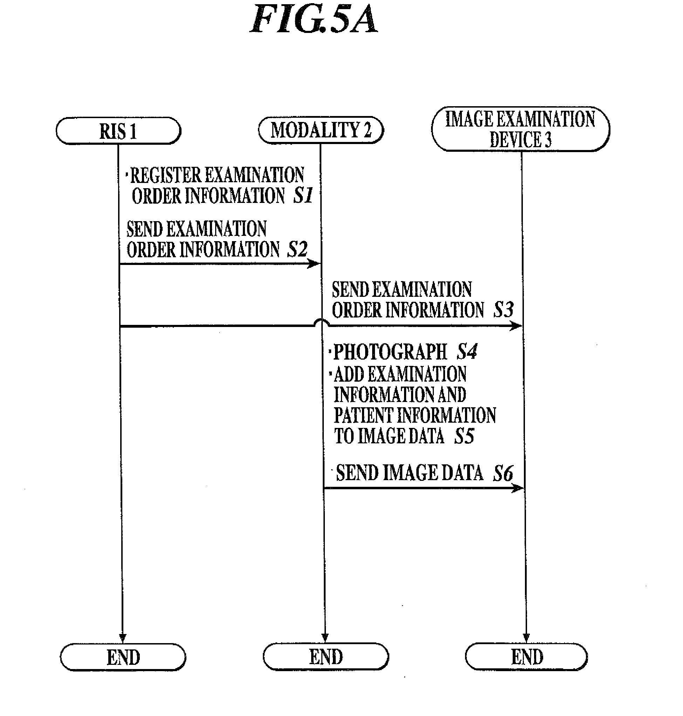

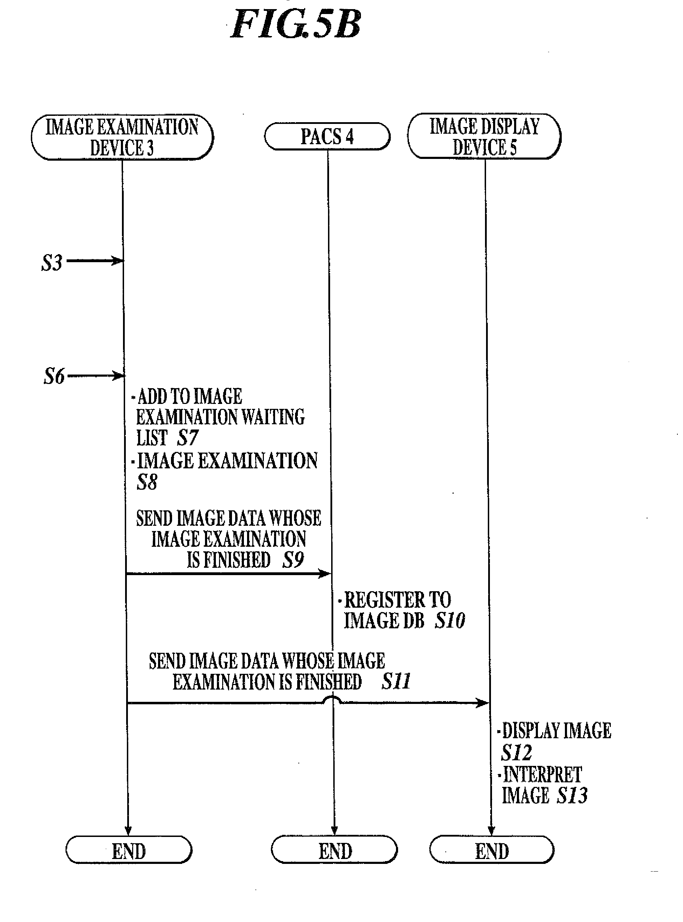

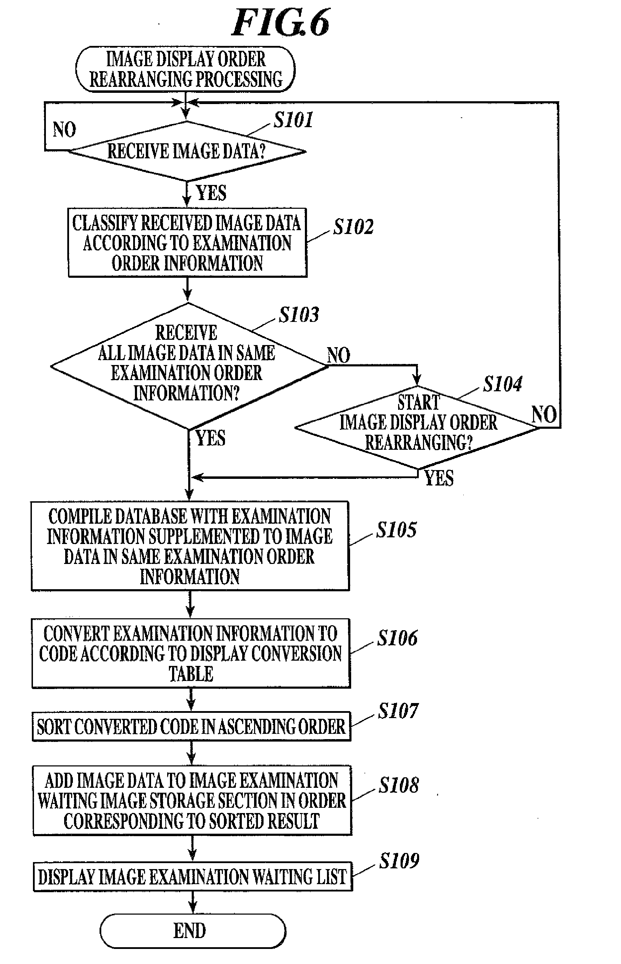

The CPU 31 reads out various programs such as a system program, processing program, or the like stored in the storage section 36 to be developed to the RAM 35, and according to the developed program, performs various processing such as processing in image examination device 3 shown in FIG. 5A to FIG. 5B or image display order rearranging processing shown in FIG. 6.

The input section 32 includes a keyboard including a cursor key, numeral input keys, various function keys, etc. and a pointing device such as a mouse, etc., and outputs to the CPU 31 a pressed signal of a key pressed down on the keyboard and operation signal of the mouse as input signals.

The display section 33 includes a monitor such as Cathode Ray Tube (CRT), Liquid Crystal Display (LCD), or the like, and displays various screens according to an instruction of a display signal input from the CPU 31.

The communication section 34 is configured with a network interface or the like and sends and receives data to and from an external device connected to the communication network N. In other words, the communication section 34 functions as a receiving member.

The RAM 35 forms a work area for temporarily storing various programs read out from the storage section 36 which can be performed by the CPU 31, input or output data, parameter, etc., in the various processing controlled by the CPU 31. The RAM 35 forms an examination order information area 351 for storing examination order information received from the RIS 1 and an image area 352 for storing image data of medical images received from the modality 2.

The storage section 36 is configured with a Hard Disk Drive (HDD), semiconductor non-volatile memory or the like. The storage section 36 stores various programs such as a system program performed by the CPU 31 or image display order rearranging processing program.

As a conversion table storage member, the storage section 36 stores a display conversion table 361 for converting examination information included in the supplementary information of the medical image sent from the modality 2 to a code for rearranging in image display order. The code for rearranging in image display order includes a combination of code for specifying "modality", code for specifying "procedure", code for specifying "site (photograph site)", code for specifying "left and right", code for specifying "body position" and code for specifying "photograph direction". In the present embodiment, as a code for rearranging in image display order, JJ1017 Ver. 3.0 code, which is being standardized for compatibility between RIS/HIS and modality, is used.

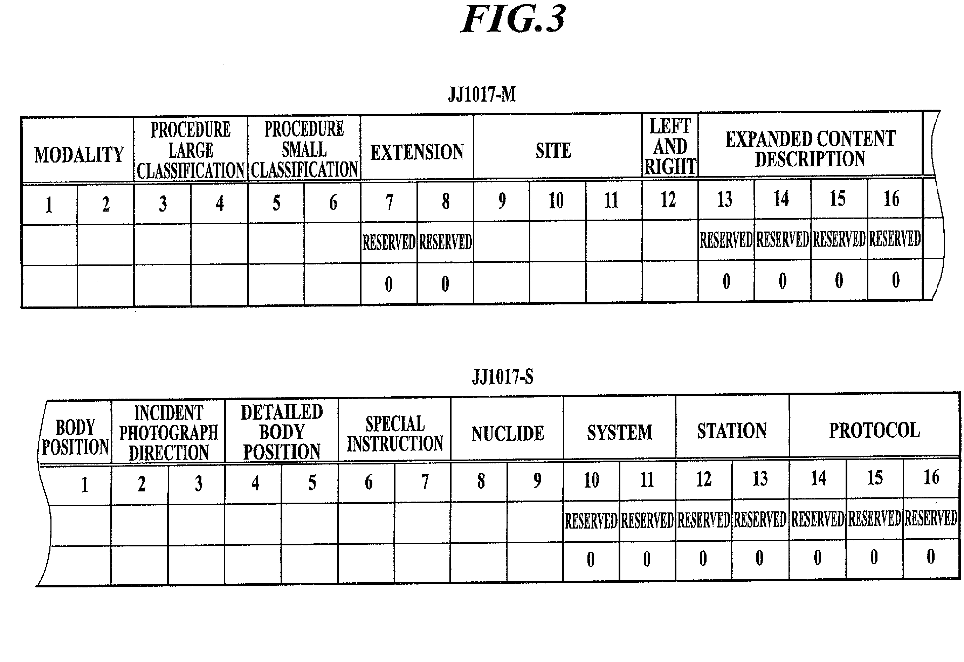

FIG. 3 shows a data structure of JJ1017 Ver. 3.0 code. In FIG. 3, numbers represent digit number and characters above the numbers represent information shown by a code of each digit. As shown in FIG. 3, JJ1017 Ver. 3.0 code is a 32 digit code, and in 16 digits of the first half (JJ1017-M), a code for specifying "modality" is placed in the first to second digits, a code for specifying a large classification of "procedure" is placed in the third to fourth digits, a code for specifying a small classification of "procedure" is placed in the fifth to sixth digits, a code for specifying "site" is placed in the ninth to eleventh digits, a code for specifying "left and right" is placed in the twelfth digit, and in 16 digits of the latter half (JJ1017-S), a code for specifying "body position" is placed in the first digit and a code for specifying "photograph direction" is placed in the second to third digits.

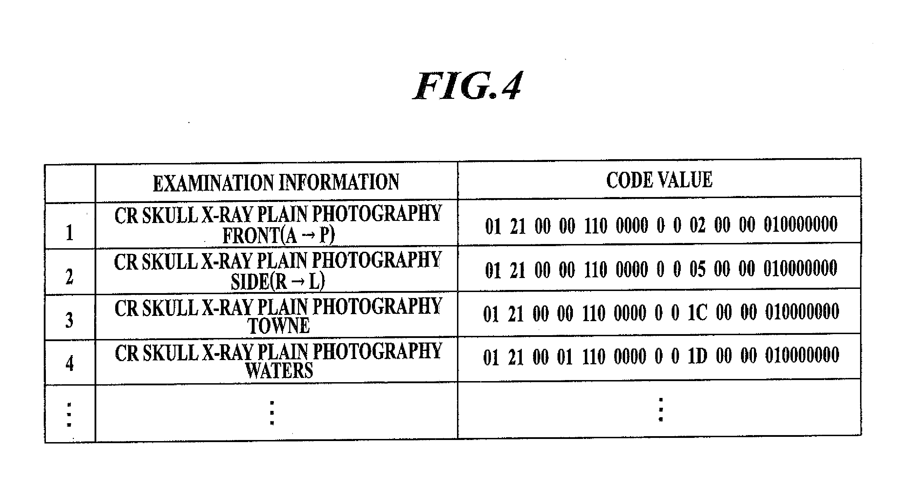

Fig. 4 shows an example of a data structure of a display conversion table 361. As shown in Fig. 4, the display conversion table 361 is a table of character string of the combination of modality information, photograph site information, photograph method information, left and right information, body position information and photograph direction information included in the examination information with corresponding code value of combination of "modality", "site", "procedure", "left and right", "body position" and "photograph direction" of code JJ1017 Ver. 3.0. Among combinations of "modality", "site", "procedure", "left and right", "body position" and "photograph direction", combination pattern used in the actual examination is fixed, thus the combination used in the actual examination is associated with the code value corresponding to the combination in the table.

The PACS 4 includes an image data base (image DB) and stores image data of the medical image and supplementary information sent from the modality 2 in the image DB. According to a request from the image display device 5, the image data is read out from the image DB and delivered to the image display device 5, which is the source of request.

The image display device 5 is a terminal device such as a personal computer (PC) provided so that a doctor can display image data of the medical image delivered from the image examination device 3 or the PACS 4 to interpret the image for diagnosis.

Next the operation will be described.

FIG. 5A to FIG. 5B are flow diagrams showing processing and flow of data among the devices configuring the medical image system 100. The processing and flow of data among the devices configuring the medical image system 100 will be described below with reference to FIG. 5A to FIG. 5B.

First, examination order information is registered and generated in the RIS 1 (step S1), and the generated examination order information is sent to the modality 2 specified in the modality information included in the examination order information as the modality to be used in photography and the image examination device 3 (step S2 and step S3).

When the modality 2 receives the examination order information from the RIS 1, photography of the photograph site of the patient is performed according to the received examination order information to generate image data of the medical image (step S4), patient information and examination information of the image data are written as supplementary information according to the examination order information in the header area of the generated image data (step S5) and the image data with the supplementary information is sent to the image examination device 3 (step S6).

When the image examination device 3 receives the examination order information sent from the RIS 1 and the image data sent from the modality 2, the received image data of the medical image is added to an image examination waiting image storage section, and a list of image examination waiting image (image examination waiting list) stored in the image examination waiting image storage section 362 is displayed on the display section 33 (step S7). When a medical image which is to be an object of image examination is selected from the image examination waiting list by the input section 32, an image examination screen including the selected medical image, supplementary information and corresponding examination order information is displayed and image examination by the image examiner is performed (step S8). Image examination is where the image examiner refers to the image examination screen to check the quality and positioning of the medical image or the consistency between the examination order information corresponding to the medical image and the supplementary information of the medical image, and when necessary, performs image density correction, contrast tuning and correction of supplementary information on the image examination screen with the input section 32.

The image data (including supplementary information) after image examination is sent to the PACS 4 (step S9), and stored in the image DB (step S10). Alternatively, the image data after image examination is sent to the image display device 5 (step S11) and displayed on the display screen (step S12) to be interpreted for diagnosis by a doctor (step S13).

As described above, examination order information registered and generated by the RIS 1 may include a plurality of examination information, and in such a case, a plurality of image data of medical images are generated by the modality 2. Also, as in examination order information including photography by the CT device 2a, CR device 2b and MRI device 2c, image data by a plurality of modalities 2 may be generated by one examination order information. Before, when the image examination device 3 or the PACS 4 or image display device 5 in a system not provided with the image examination device 3 received image data from the modality 2, the images (or image list) were displayed in the order received.

However, although the pattern of the series of examinations specified by the examination order information is fixed to some extent in each medical facility according to a site to be examined or a case, a plurality of modalities 2 may perform photography in one examination order information, there are many occasions where a patient comes and goes between a plurality of modalities or a plurality of modalities photograph a plurality of patients at the same time, and thus even if the examination pattern is fixed, the order of examination is often different at different times. Thus, when the images are displayed in the order the image data is received (in other words, the order of examination), when a series of images photographed in the same examination pattern are displayed, the image display order is not in a predetermined order, and moreover, images of different patients may be mixed. Thus, before checking the image itself, an image examiner or a doctor who checks the images needs to change the image display order while checking the examination order information in order to confirm whether or not all examinations in one examination order information are performed or to enhance efficiency of image checking (image examination), and thus excess work is necessary which is troublesome.

In the present embodiment, the image examination device 3 performs the image display order rearranging processing shown in FIG. 6 to rearrange and display image data received from the modality 2 for one examination data information in an order predetermined in the device so that the image examiner does not need to rearrange the display order of the images before checking the images and consequently, image examination can be performed efficiently.

The image display order rearranging processing will be described below with respect to FIG. 6. The processing is realized with software processing by the CPU 31 in coordination with the program of the image display order rearranging processing stored in the storage section 36, and sort, control and code conversion are realized by performing the processing.

As a previous step to the processing, when examination order information is received from the RIS 1 through the communication section 34, the CPU 31 stores the received examination order information in the examination order information area 351 and also reserves an image area in the image area 352 for storing image data of the examination order information, in other words, image data with the same examination ID as the examination order information.

When the image examination device 3 receives image data of the medical image from the modality 2 by the communication section 34 (step S101), the supplementary information of the received image data is referred to, and the received image data is classified according to the examination order information based on the examination ID included in the supplementary information (step S102). In other words, the image data is stored in the image area reserved for examination order information corresponding to the received image data. Next, it is judged whether or not all image data corresponding to the same examination order information as that of the received image data is received according to examination information included in the examination order information corresponding to the received image data stored in the examination order information area 351 and the supplementary information of the image data stored in the image area where the received image data is classified (step S103), and when it is judged that all image data is received (step S103; YES), the processing advances to step S105.

When it is judged that all image data of the same examination order information is not yet received (step S103; NO), it is judged whether or not an image display order rearranging instruction is input from the input section 32, and when the image display order rearranging instruction is not input (step S104; NO), the processing returns to step S101. When the image display order rearranging is instructed from the input section 32 (step S104; YES), the processing advances to step S105. It is preferable that the display section 33 displays, for example, an icon for instructing display of status of receiving image data corresponding to the examination order information and when the display of the status of the receiving is instructed by the image examiner pressing down the icon with the input section 32, the examination order information of the image data which started to be received and the time the image data started to be received are displayed on the display section 33. With this, the image examiner can instruct rearranging in the image display order from the input section 32 and proceed with the image examination when it is determined that the image examination should proceed, even when all image data is not received, according to the time the receiving started (for example, when the receiving does not finish even after n minutes or more (n is to be a positive value) pass from the time the receiving started).

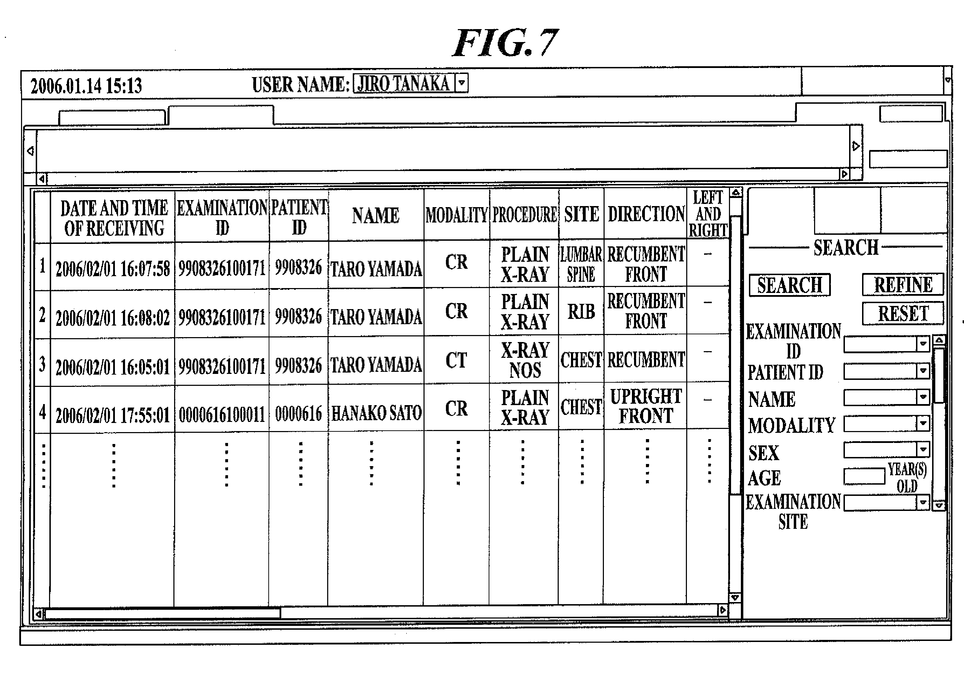

In step S105, the examination information included in the supplementary information of the image data photographed according to the same examination order information is obtained and read out to the work area of the RAM 35 to compile a database (step S105). Next the display conversion table 361 as shown in FIG. 4 is referred to so as to convert the examination information to a code according to the display conversion table 361 (step S106) and the converted code is sorted (rearranged) in ascending order (step S107). Next, the image data is assigned with an image examination waiting list number in the order corresponding to the sorted result and the image data is successively registered additionally in the image examination waiting image storage section 362 in the order corresponding to the sorted result (step S108). When the image data is added to the image examination waiting image storage section 362, the supplementary information of the image data stored in the image examination waiting image storage section 362 is obtained, an image examination waiting list is generated by listing the obtained supplementary information in the order of the image examination waiting list number to be displayed on the display section 33 (step S109) and the processing ends. FIG. 7 shows an example of a screen displaying the image examination waiting list. As shown in FIG. 7, the image examination waiting list is not displayed in the order the image data is received, but displayed in the ascending order of the code defined by the display conversion table 361.

After the image display order rearranging processing, the following processing is performed by the CPU 31 in coordination with a program stored in the storage section 36 according to an operation by the image examiner. In other words, when the medical image which is the object of image examination is selected by the image examiner with the input section 32 in the order of the image examination waiting list, the image data of the selected medical image is read out from the image examination waiting image storage section 362 and an image examination screen (not shown) for examination of the selected medical image is displayed on the display section 33. The image examination screen displays the selected medical image with the supplementary information of the medical image and the examination order information received from the RIS 1. The adjustment buttons for adjusting the density and contrast of the displayed medical image are displayed on the image examination screen, and when the image examiner operates the adjustment buttons with the input section 32, image processing is performed on the displayed medical image according to the operation. Also, the displayed supplementary information can be corrected on the image examination screen, thus when the content of the examination order information and the supplementary information do not match, the correct information is input from the input section 32 by the image examiner to correct the supplementary information.

The image examination screen may display a series of medical images photographed according to the same examination order information on one screen. In this case also, the medical images of the same examination order information are displayed in the order of image examination waiting list number, in other words, in the ascending order of the code defined by the display conversion table 361.

As described above, with the image examination device 3, the image data received from the modality 2 is classified according to the examination order information based on the examination ID, the examination information included in the supplementary information of the image data photographed according to the same examination order information is obtained, the obtained examination information is converted to a code according to the display conversion table 361, and the converted code is sorted in ascending order. The image data is assigned with an image examination waiting list number in the order corresponding to the sorted result to be successively registered additionally in the image examination waiting image storage section 362 and an image examination waiting list listing the supplementary information of the image data stored in the image examination waiting image storage section 362 in the order of the image examination waiting list number is generated and displayed on the display section 33.

Thus, since the series of medical images according to examination order information and the list thereof are displayed in a predetermined order, the series of medical images photographed in the predetermined examination pattern or the list thereof is constantly displayed in a predetermined order, thus the image examiner who performs checking by displaying the photographed medical image can easily know whether or not all examinations for one examination order information have been performed without changing the display order of the medical images or the list, and the checking of the series of images can be performed in a predetermined order, and consequently, the operation efficiency can be enhanced. Also, when there is a failure among examinations in the examination order, the image examiner can promptly specify which examination was not performed so that the image examiner can urge the photography technician to perform photography of the examination which was not performed, and thus the burden of both the photography technician and the patient, such as performing photography of the examination which was not performed at a later date, can be reduced.

### [Second Embodiment]

The second embodiment will be described below with reference to the drawings.

First, a structure of the second embodiment will be described.

In the second embodiment, a structure of the display conversion table stored in the storage section 36 is different from the structure described in the first embodiment. In the present embodiment, the storage section 36 stores a display conversion table 364 as a sorting order table storage member.

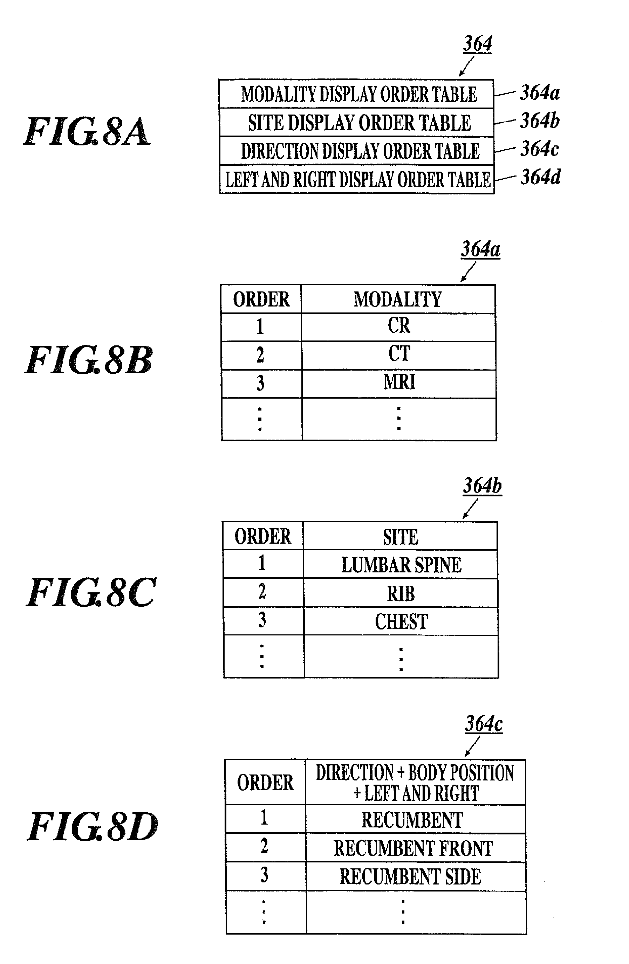

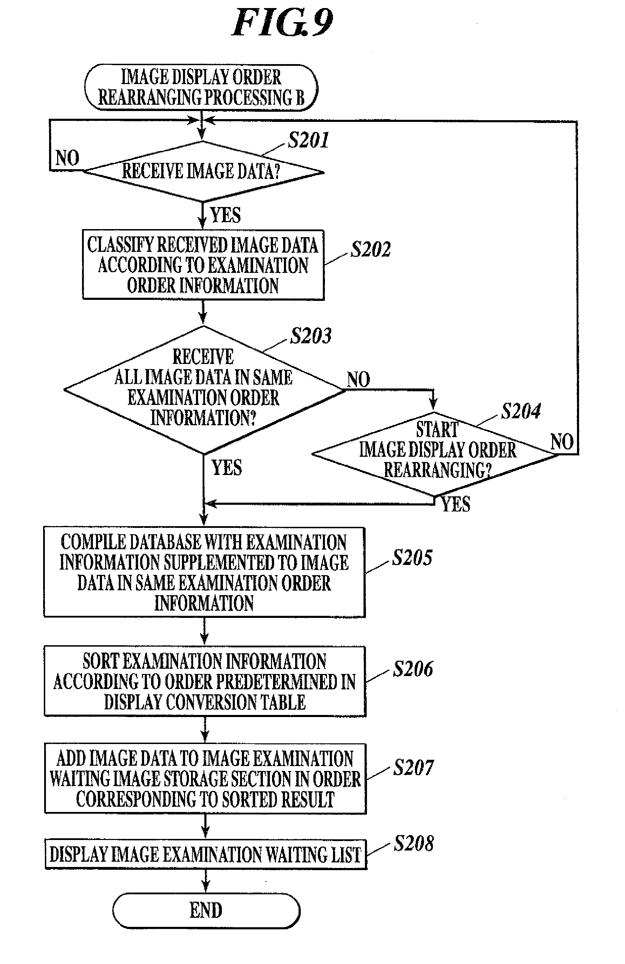

FIG. 8A shows an example of a data structure of the display conversion table 364. As shown in FIG. 8A, in the second embodiment, the display conversion table 364 includes a modality display order table 364a (see FIG. 8B), a site display order table 364b (see FIG. 8C), a direction display order table 364c (see FIG. 8D), and the like. The modality display order table 364a is a table where the modality information is arranged from a higher order of the sorted order in an image display order rearranging processing B (see FIG. 9). The site display order table 364b is a table where the photograph site information is arranged from a higher order of the sorted order in an image display order rearranging processing B. The direction display order table 364c is a table where a combination of information including at least one of photograph direction information, body position information and left and right information is arranged from a higher order of the sorted order in an image display order rearranging processing B. The priority order of the tables is modality display order > site display order > direction display order.

Other structures are similar to those described in the first embodiment, thus description is omitted.

Next, the operation of the second embodiment will be described.

Processing and flow of data of the devices in the second embodiment are similar to those described in the first embodiment, thus the description is omitted, and the image display order rearranging processing whose operation is different from the operation described in the first embodiment due to the different display conversion table will be described.

FIG. 9 shows the image display order rearranging processing B of the second embodiment. The image display order rearranging processing B will be described below with reference to FIG. 9. The processing is realized with software processing by the CPU 31 in coordination with the program of the image display order rearranging processing B stored in the storage section 36, and sort and control are realized by performing the processing.

When the image examination device 3 receives image data of the medical image from the modality 2 by the communication section 34 (step S201), the supplementary information of the received image data is referred to, and the received image data is classified according to the examination order information based on the examination ID included in the supplementary information (step S202). In other words, the image data is stored in the image area reserved for examination order information corresponding to the received image data. Next, it is judged whether or not all image data corresponding to the same examination order information as that of the received image data is received according to examination information included in the examination order information corresponding to the received image data stored in the examination order information area 351 and the supplementary information of the image data stored in the image area where the received image data is classified (step S203), and when it is judged that all image data is received (step S203; YES), the processing advances to step S205.

When it is judged that all image data of the same examination order information is not yet received (step S203; NO), it is judged whether or not an image display order rearranging instruction is input from the input section 32, and when the image display order rearranging instruction is not input (step S204; NO), the processing returns to step S201. When the image display order rearranging is instructed from the input section 32 (step S204; YES), the processing advances to step S205. It is preferable that the display section 33 displays, for example, an icon for instructing display of status of receiving image data corresponding to the examination order information and when the display of the status of the receiving is instructed by the image examiner pressing down the icon with the input section 32, the examination order information of the image data which started to be received and the time the image data started to be received are displayed on the display section 33. With this, the image examiner can instruct rearranging in the image display order from the input section 32 and proceed with the image examination when it is determined that the image examination should proceed, even when all image data is not received, according to the time the receiving started (for example, when the receiving does not finish even after n minutes or more (n is to be a positive value) pass from the time the receiving started).

In step S205, among the examination information included in the supplementary information of the image data photographed according to the same examination order information, the modality information, the photograph site information, the photograph direction information, the body position information and the left and right information are obtained and read out to the work area of the RAM 35 to be compiled as a database (step S205). Next, the display conversion table 364 is referred to so as to sort the read out examination information according to an order defined by the display conversion table 364 (step S206). Next, the image data is assigned with an image examination waiting list number in the order corresponding to the sorted result and the image data is successively registered additionally in the image examination waiting image storage section 362 in the order corresponding to the sorted result (step S207). When all of the image data of the same examination order information is added to the image examination waiting image storage section 362, the supplementary information of the image data stored in the image examination waiting image storage section 362 is obtained, an image examination waiting list is generated by listing the obtained supplementary information in the order of the image examination waiting list number to be displayed on the display section 33 (step S208) and the processing ends.

The processing performed by the image examination device 3 after the image display order rearranging processing B is similar to that described in the first embodiment. The image examination screen may display a series of medical images photographed according to the same examination order information on one screen. In this case also, the medical images of the same examination order information are displayed in the order of image examination waiting list number, in other words, in the order defined by the display conversion table 364.

As described above, with the image examination device 3, the image data received from the modality 2 is classified according to the examination order information based on the examination ID, the examination information included in the supplementary information of the image data photographed according to the same examination order information is obtained, and the obtained examination information is sorted according to the display conversion table 364. The image data is assigned with an image examination waiting list number in the order corresponding to the sorted result to be successively registered additionally in the image examination waiting image storage section 362 and an image examination waiting list listing the supplementary information of the image data stored in the image examination waiting image storage section 362 in the order of the image examination waiting list number is generated and displayed on the display section 33.

Thus, since the series of medical images according to examination order information and the list thereof are displayed in a predetermined order, the series of medical images photographed in the predetermined examination pattern or the list thereof is constantly displayed in a predetermined order, thus the image examiner who performs checking by displaying the photographed medical image can easily know whether or not all examinations for one examination order information have been performed without changing the display order of the medical images or the list, and the checking of the series of images can be performed in a predetermined order, and consequently, the operation efficiency can be enhanced. Also, when there is a failure among examinations in the examination order, the image examiner can promptly specify which examination was not performed so that the image examiner can urge the photography technician to perform photography of the examination which was not performed, and thus the burden of both the photography technician and the patient, such as performing photography of the examination which was not performed at a later date, can be reduced.

The descriptions of the above-described embodiments are suitable examples of the medical image system 100 of the present embodiment, and the present invention is not limited to the embodiments shown.

For example, the medical image system 100 includes the RIS 1 to image display device 5, however the system may be connected to a printer, etc., and after the image examination is finished, the image data may be sent to the printer to output the medical image from the printer.

The detailed structure and the detailed operation of the devices configuring the medical image system 100 may be suitably modified without leaving the spirit of the present invention.

The disclosure of Japanese Patent Application No. 2006-105321 filed on April 6, 2006 to the Japanese Patent Office including description, claims, figures and abstract is incorporated herein by reference in its entirety.

### Industrial Applicability

The present invention may be applied to the field of medicine, in a medical information processing device for checking and correcting a medical image generated in an image generation device and supplementary information.

### Description of Reference Numerals

100 medical image system
1 RIS
2 modality
2a CT device
2b CR device
2c MRI device
3 image examination device
4 PACS
5 image display device
31 CPU
32 input section
33 display section
34 communication section
35 RAM
351 examination order information area
352 image area
36 storage section
361 display conversion table
362 image examination waiting image storage section
364 display conversion table
37 bus
N communication network

## Claims

1. A medical information processing device connected to one or more image generation device which generates a medical image based on examination order information and supplements to the generated medical image supplementary information including identification information for identifying the examination order information, and patient information and examination information concerning the medical image, the medical information processing device including a display section to display the medical image generated by the image generation device, the medical information processing device comprising:
a receiving section to receive the medical image generated by the one or more image generation device and to receive the supplementary information of the medical image;
a sorting section to classify the medical image received by the receiving section with respect to medical image generated based on the examination order information and to sort the examination information supplemented to the medical image generated based on a same examination order information in a predetermined order; and
a control section to allow the display section to display a series of the medical image generated based on the same examination order information by the one or more image generation device or to display a list of the series of medical image.

2. The medical information processing device of claim 1, further comprising:
a conversion table storage section which stores a conversion table for converting the examination information into a code representing the examination information; and
a code conversion section to convert the examination information supplemented to the medical image generated based on the same examination order information according to the conversion table, wherein
the sorting section sorts the examination information by sorting in ascending order the code resulting from the conversion by the code conversion section.

3. The medical information processing device of claim 1, wherein
the examination information includes image generation device information, photograph site information, photograph direction information, body position information and left and right information to specify content of the examination;
the medical information processing device includes a sorting order table storage section to store a table showing a sorting order of the image generation device information, a table showing a sorting order of the photograph site information, a table showing a sorting order of a combination of the photograph direction information, body position information and left and right information; and
the sorting section sorts the examination information supplemented to the medical image generated based on the same examination order information according to the table stored in the sorting order table storage section.
